# FASCICULE DE BREVET EUROPEEN

(11) **EP 0 664 290 B1**
(45) Date de publication et mention de la délivrance du brevet: **03.11.1999**
(21) Numéro de dépôt: 95400115.2
(22) Date de dépôt: 20.01.1995
(51) Int. Cl.: C07D 307/62, A61K 7/00, A61K 31/34, A23L 3/34

(54) **Mono-et di-esters d'acide cinnamique ou de l'un de ses dérivés et de vitamine C, leur procédé de préparation et leur utilisation comme anti-oxydants dans des compositions cosmétiques, pharmaceutiques ou alimentaires**
Zimtsäure mono- und di-Ester oder Ester von Derivaten und Vitamin C, Verfahren zu deren Herstellung und deren Verwendung als Antioxidantien in kosmetischen, pharmazeutischen oder Nahrungszusammensetzungen
Mono- and di-esters of cinnamic acid or of one of his derivatives and of vitamine C, their process of preparation and their use as antioxydants in cosmetic, pharmaceutical or alimentary compositions

(30) Priorité: 20.01.1994 FR 9400583
(43) Date de publication de la demande: 26.07.1995
(73) Titulaire: L'OREAL, 75008 Paris (FR)
(72) Inventeur: Galey, Jean-Baptiste, F-93600 Aulnay-sous-Bois (FR); Terranova, Eric, F-92600 Asnieres (FR)
(74) Mandataire: Stalla-Bourdillon, Bernard

(56) Documents cités:
- EP-A- 0 074 411
- EP-A- 0 146 121
- EP-A- 0 202 589
- BE-A- 1 000 307
- FR-A- 1 196 898
- FR-A- 1 374 746
- FR-A- 1 530 414
- FR-A- 2 580 644
- DATABASE WPI Week 988 9 Mai 1984 Derwent Publications Ltd., London, GB; AN 54 'ANTIOXIDANT' & JP-A-59 015 477 (TANABE SEIYAKU K.K.) , 16 Juillet 1982

## Description

La présente invention a pour objet de nouveaux mono- et di-esters d'acide cinnamique ou de l'un de ses dérivés et de vitamine C, leur procédé de préparation et leur utilisation en tant que substances anti-oxydantes dans des compositions cosmétiques, ou pharmaceutiques, en vue non seulement de les protéger contre l'oxydation, mais également de leur conférer des propriétés protectrices à l'égard de l'oxydation des constituants lipidiques de la peau.

Comme ceci est bien connu la vitamine C ou acide ascorbique est un produit naturel que l'on rencontre dans de nombreux fruits ou légumes, celle-ci présentant des propriétés anti-scorbutiques dans la mesure où elle participe à la synthèse du collagène qui est le constituant principal des fibres protéiques du tissu humain.

La vitamine C permet en effet d'hydroxyler deux acides aminés, la lysine et la proline, en maintenant le fer du cofacteur de la lysine hydroxylase et de la proline hydroxylase à l'état réduit Fe²+.

Par ailleurs, la vitamine C intervient également dans d'autres processus d'oxydation et d'hydroxylation enzymatique en particulier dans l'hydroxylation de la dopamine en noradrénaline catalysée par la dopamine β-hydroxylase ou encore dans celle du tryptophane en 5-hydroxy tryptophane catalysée par la tryptophane hydroxylase.

La vitamine C présente en outre des propriétés réductrices qui lui confèrent un rôle d'anti-oxydant naturel, décrites dans la littérature, notamment dans la revue de G.W. Burton et Col., Adv. in Free Radical Biology and Medicine, 419 (1986).

Si la vitamine C comme on vient de l'indiquer possède de nombreuses propriétés biologiques indispensables, elle n'est pas toutefois sans présenter certains inconvénients dans la mesure où elle est auto-oxydable, thermosensible et instable "*in vitro*" en milieu aqueux notamment en pH alcalin.

En vue de remédier à ce phénomène d'auto-oxydation il a été envisagé par plusieurs auteurs de stabiliser la vitamine C selon différents moyens.

Parmi ceux-ci on peut mentionner celui consistant à créer une association chimique de l'acide ascorbique avec par exemple l'acide gluconique ou l'acide urocanique.

Un autre moyen a consisté à stabiliser la vitamine C par des méthodes physiques en l'incorporant par exemple dans des cyclodextrines, des zéolithes ou encore dans les liposomes.

Il a en outre été proposé d'effectuer des dérivés de la vitamine C, plus particulièrement sous forme de phosphodiesters avec la vitamine E par exemple.

Enfin un dernier moyen de stabilisation a été suggéré, celui-ci consistant à fonctionnaliser chimiquement le groupement ènediol de la vitamine C par formation d'une fonction phosphate, sulfate ou par formation d'une fonction éther ou ester.

A cet égard, de nombreux auteurs ont proposé des monoesters en position 2 de la vitamine C en vue de la stabiliser, ceux-ci étant essentiellement des alkyl esters ayant de 1 à 18 atomes de carbone, des fluoroalkyl esters ayant de 2 à 7 atomes de carbone et de 4 à 15 atomes de fluor, ou encore des aryl esters et en particulier des benzoates éventuellement substitués (voir par exemple FR-A-1530414, FR-A-1374746, FR-A-1196898).

On a maintenant constaté de façon surprenante que les esters de l'acide cinnamique ou de l'un de ses dérivés et de vitamine C étaient beaucoup plus stables à l'auto-oxydation, non seulement que la vitamine C, mais également que certains de ses dérivés déjà décrits dans la littérature. De plus, on a constaté de façon inattendue que les composés selon l'invention étaient beaucoup plus efficaces que la vitamine C et ses dérivés vis-à-vis de la protection des cellules épidermiques soumises à l'action de radicaux libres.

L'intérêt principal des mono- et di-esters de vitamine C selon l'invention, réside dans le fait que le groupement acyle de ces esters dérive essentiellement de la structure d'un produit naturel à savoir l'acide cinnamique, ou de l'un de ses dérivés tels que l'acide férulique, l'acide caféique ou l'acide sinapique ceux-ci présentant en eux-mêmes des propriétés anti-oxydantes.

Il s'est avéré en outre, que les esters de vitamine C selon l'invention présentent un caractère lipophile beaucoup plus prononcé que celui de la vitamine C, ce qui leur permet une meilleure pénétration dans le stratum cornéum.

Par ailleurs, les esters de vitamine C selon l'invention peuvent être considérés comme d'excellents véhicules de la vitamine C dans la mesure où en présence d'enzymes épidermiques, du type estérase, ils peuvent s'hydrolyser et libérer ainsi la vitamine C.

La présente invention a donc pour objet à titre de composés nouveaux des mono- et di-esters d'acide cinnamique ou de l'un de ses dérivés et de vitamine C répondant à la formule générale suivante : 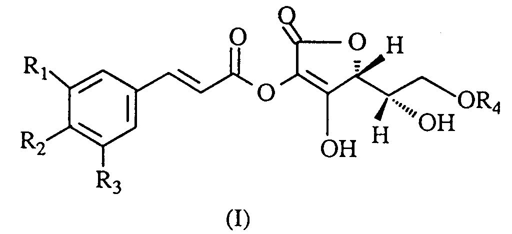 dans laquelle :
R₁, R₂ et R₃, identiques ou différents, représentent un atome d'hydrogène, un radical hydroxy, alkoxy, fluoroalkoxy ou alkylcarbonyloxy, et
R₄ représente un atome d'hydrogène ou -COR₅, R₅ représentant un radical alkyle en C₁-C₂₀ ou le radical de formule :

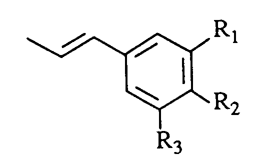

Selon l'invention, le radical alkoxy est de préférence un radical méthoxy, éthoxy ou butoxy ; le radical fluoroalkoxy est de préférence un radical trifluorométhoxy et le radical alkylcarbonyloxy est de préférence un radical acétoxy, propionyloxy ou butyryloxy.

Parmi les composés de formule (I) selon l'invention, on peut en particulier citer :
- le 2-O-cinnamate d'ascorbyle,
- le 2-O-férulate d'ascorbyle,
- le 2-O-caféate d'ascorbyle,
- le 2-O-sinapate d'ascorbyle, et
- le 4'-acétoxy férulate de 2-O-[6-palmitoyl ascorbyle].

La synthèse des composés selon l'invention peut être facilement réalisée selon le schéma réactionnel suivant :

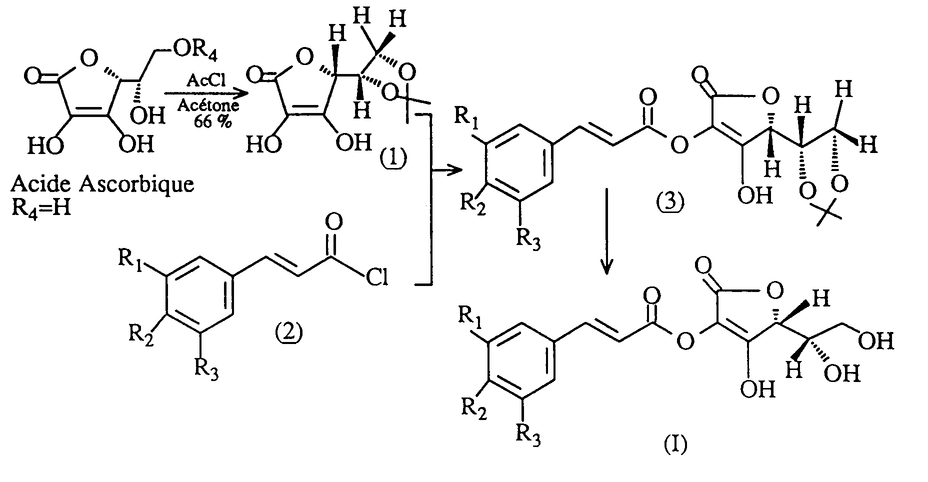

Lorsque R₄ représente un atome d'hydrogène, la première étape de préparation des mono-esters consiste à protéger les fonctions hydroxy en position 5 et 6 de l'acide ascorbique par formation d'un cétal cyclique du type 5,6-O-isopropylidène obtenu par réaction de l'acide ascorbique dans l'acétone en présence de chlorure d'acétyle en tant que catalyseur. L'acide 5,6-O-isopropylidène ascorbique est obtenu avec un rendement d'environ 65-70 % et se présente sous forme d'un solide blanc cotoneux.

Le chlorure d'acide de formule (2) peut être commercial tel que le chlorure de cinnamoyle ou obtenu par synthèse lorsqu'il comporte des substituants sur le noyau aromatique ou encore à partir de certains acides commerciaux tels que par exemple l'acide férulique.

Toutefois, lorsque l'un des substituants R₁, R₂ ou R₃ est un groupement hydroxy, celui-ci doit être préalablement protégé par exemple par acétylation de l'acide aromatique hydroxylé correspondant en présence d'anhydride acétique et d'une base telle que par exemple la 4-N,N-diméthylaminopyridine.

Le chlorure d'acide est alors préparé par réaction de l'acide aromatique acétylé avec du chlorure de thionyle dans un solvant organique tel que le toluène à une température comprise entre 50 et 120°C.

La réaction entre le chlorure d'acide (2) et l'acide 5,6-O-isopropylidène ascorbique (1) est réalisée dans un solvant organique tel que le dichlorométhane en présence d'imidazole ce qui conduit à l'ester attendu (3) avec un rendement d'environ 90 %.

Le groupe protecteur des fonctions 5,6-dihydroxy de l'acide ascorbique et éventuellement de la ou des fonction(s) hydroxy du noyau aromatique est alors éliminé par agitation du mono-ester obtenu (3) dans l'acétone en présence d'HCl 1N à 3N.

Lorsque R₄, dans la formule (I) représente le radical -COR₅ les composés sous forme de di-ester sont tout d'abord préparés par acylation en position 6 de l'acide ascorbique selon les méthodes conventionnelles puis l'on fait ensuite réagir comme ci-dessus, l'ester d'ascorbyle obtenu avec un chlorure d'acide de formule (2).

Certains esters d'ascorbyle en position 6 sont commerciaux tels que par exemple le 6-O-palmitate d'ascorbyle.

L'efficacité des mono- et di-esters selon l'invention a été étudiée *in vitro* en utilisant des kératinocytes d'épidermes humains en primo culture, soumis à l'action d'un stress oxydant, comme par exemple le système hypoxanthine/xanthine oxydase, l'action des UV, ou encore l'addition de sels de fer.

Le stress oxydant conduit à un déséquilibre de la balance anti-oxydant/pro-oxydant par formation de radicaux libres oxygénés comme l'anion superoxyde ou le radical hydroxyle. L'action de ces radicaux libres se traduit par des dégâts oxydatifs sur les constituants cellulaires, en particulier les membranes cellulaires, ce qui entraîne la mort des cellules.

Le pouvoir protecteur des composés selon l'invention a été étudié à différentes concentrations en mesurant le pourcentage de cellules encore vivantes 1 h après le stress oxydant.

Les résultats obtenus ont permis de mettre en évidence que les composés selon l'invention avaient une excellente activité protectrice à des concentrations faibles par rapport à celles des composés couramment utilisés dans les produits cosmétiques et pharmaceutiques tels que l'acide ascorbique et ses principaux dérivés décrits dans la littérature comme le 2-benzoate d'ascorbyle ou l'acide 2-O-glucopyranosyl ascorbique. Ainsi, pour le système hypoxanthine/xanthine oxydase, les kératinocytes sont protégés par des concentrations micromolaires des composés selon l'invention alors que pour avoir une protection équivalente, il est nécessaire d'utiliser plus de 1000 fois plus de vitamine C libre.

Bien que le mécanisme d'action n'ait pu être mis en évidence de façon certaine, on suppose qu'il est soit indirect (pouvoir réducteur) soit direct (piégeage des radicaux libres).

La présente invention a également pour objet des compositions cosmétiques, pharmaceutiques ou alimentaires contenant des corps gras, caractérisées par le fait qu'elles renferment au moins un mono- ou di-ester de vitamine C de formule (I) tel que défini précédemment en tant qu'agent anti-oxydant.

Les corps gras présents dans les compositions de l'invention sont par exemple des corps gras d'origine animale, tels que l'acétine (blanc de baleine), la cire d'abeilles, la lanoline, le perhydrosqualène, l'huile de tortue, etc. des corps gras végétaux sous forme d'huile, de graisses ou de cires, telle que l'huile d'amandes douces, l'huile d'avocat, l'huile d'olive, l'huile de sésame, l'huile de macadamia les huiles de coprah ou de palme hydrogénée, le beurre de cacao, la cire de carnauba, la cire de montana ainsi que des huiles synthétiques constituées par des esters et/ou éthers de glycérol ou de glycol tels que par exemple ceux décrits dans FR-B 75.24656 (2.281.916), 75.24657 (2.281.743) et 75.24658 (2.281.744).

En plus des corps gras plus ou moins oxydables, les compositions cosmétiques ou pharmaceutiques peuvent contenir des produits sensibles à l'oxydation tels que par exemple, de la vitamine F ou du β-carotène.

Les compositions selon l'invention peuvent se présenter sous forme d'une solution huileuse, d'une émulsion eau-dans-l'huile ou huile-dans-l'eau, d'un produit solide éventuellement anhydre, d'une lotion ou d'une microdispersion, d'une dispersion réticulaire, de lipide constitutif de vésicules pouvant être du type ionique ou non ionique, ou bien un mélange de ceux-ci. Elles constituent également des laits pour le soin de la peau, des crèmes (crème pour le visage, pour les mains, pour le corps, crème anti-solaire, crème démaquillante, crème de fond de teint), des fonds de teint fluides, des laits démaquillants, des laits anti-solaires, des huiles pour le bain, des rouges à lèvres, des fards à paupière, des sticks déodorants, etc.

Pour l'application par voie topique les compositions pharmaceutiques selon l'invention comprennent des véhicules et ingrédients nécessaires pour permettre de présenter les compositions par exemple sous forme d'un onguent, d'une crème, d'un lait, d'une pommade ou d'une solution huileuse.

Selon une forme de réalisation préférée, les compositions cosmétiques ou dermopharmaceutiques, se présentent sous une forme destinée à être appliquées par voie topique en particulier de crèmes destinées à la protection de l'oxydation des lipides de la peau.

Dans les compositions selon l'invention les mono- et di-esters de vitamine C de formule générale (I) telle que définie ci-dessus, sont généralement présents en une proportion comprise entre 0,01 et 5 % en poids par rapport au poids total de la composition.

Les compositions selon l'invention peuvent en outre contenir des composés actifs ou des ingrédients utilisés de façon usuelle dans les compositions mentionnées ci-dessus, tels que des agents tensio-actifs, des colorants, des parfums, des produits astringents, des produits absorbant les ultra-violets, des solvants organiques. Ces compositions sont préparées selon les méthodes usuelles.

On va maintenant donner à titre d'illustration plusieurs exemples de préparation des mono- ou di-esters d'acide cinnamique ou de l'un de ses dérivés et de vitamine C de formule générale (I) ainsi que des exemples de composition cosmétique et pharmaceutique à base de ceux-ci.

### EXEMPLES DE PREPARATION

### EXEMPLE 1 : Préparation du 2-O-férulate d'ascorbyle

### (1) Préparation de l'acide 4-acétoxy férulique

Dans un ballon tricol de 250 ml, équipé d'un réfrigérant, d'un thermomètre et d'une agitation magnétique, on introduit 16,4 g d'acide férulique (0,084 mole) dans 50 ml d'anhydride acétique et 0,51 g de 4-N,N-diméthylaminopyridine. Après agitation du milieu réactionnel à température ambiante pendant 3 h 45, on hydrolyse le milieu avec 100 ml d'eau.

Après une nuit d'agitation, on filtre le produit, rince à l'eau (2x100 ml) et sèche sous vide et sur P₂O₅ à température ambiante. On recueille 20 g d'acide 4-acétoxy férulique (0,084 mole) ; Rendement (Rdt) = 100 % sous forme d'une poudre blanche.
Point de fusion = 199-200°C (Köfler)
Rf. (Gel de Silice : Merck 60 F254) = 0,41 (CH₂Cl₂ : CH₃OH=9:1).
- RMN ¹H (DMSO d6 ; 200 MHz): 2,09(S ; CH₃CO) ; 3,65(S ; CH₃O) ; 6,43(d ; C=CH-CO ; J=16Hz) ; 6,94(d ; Ar-H ; J₁=8,2Hz) ; 7,09(dxd ; Ar-H ; J₁=8,2 Hz et J₂=1,4 Hz) ; 7,30(d ; Ar-H ; J₂=1,4 Hz) ; 7,74(d ; Ar-CH=C ; J=16 Hz) ; 12,24(large S ; CO-OH)
- RMN ¹³C (DMSO d6 ; 50 MHz): 20,3 ; 55,9 ; 111,8 ; 119,5 ; 121,3 ; 123,2 133,3 ; 140,8 ; 143,3 ; 151,2 ; 167,6 ; 168,4

Analyse élémentaire : C₁₂H₁₂O₅ ; Poids Moléculaire (P.M.) = 236

| | C % | H % | O % |
|---|---|---|---|
| Calculé | 61,02 | 5,09 | 33,90 |
| Trouvé | 59,92 | 5,08 | 33,94 |

### (2) Préparation du chlorure de l'acide 4-acétoxy férulique

Dans un ballon tricol de 500 ml équipé d'un thermomètre, d'une entrée d'argon, d'un réfrigérant surmonté d'une garde à chlorure de calcium et d'une agitation magnétique, on introduit 12,2 g d'acide 4-acétoxy férulique (0,051 mole) obtenu ci-dessus et 15 ml de chlorure de thionyle (d = 1,64 ; 0,204 mole) dans 200 ml de toluène. On chauffe le milieu sous argon pendant 1 h au reflux du solvant, puis chasse le toluène à l'aide d'un évaporateur rotatif. Le produit brut obtenu est soit utilisé directement dans l'étape de couplage avec l'acide 5,6-O-isopropylidène ascorbique, soit recristallisé dans 50 ml de toluène. Après 24 h de séchage sous vide et sur P₂O₅ à température ambiante, on recueille 9,32 g du chlorure de l'acide 4-acétoxy férulique (0,037 mole) ; Rdt = 72 % sous forme de cristaux jaune pâle.
Point de fusion = 139-140°C (Köfler)
- RMN ¹H (CDCl₃ ; 200 MHz): 2,24(S ; AcO) ; 3,76(S; CH₃O) ; 6,49(d ; C=CH-CO ; J=15,5 Hz) ; 6,98-7,10(m ; 3xAr-H) ; 7,68(d ; Ar-CH=C ; J=15.5 Hz);
- RMN ¹³C (CDCl₃ ; 50 MHz): 21,5 ; 56,8 ; 112,8 ; 123,2 ; 123,4 ; 124,5 132,7 ; 143,7 ; 150,8; 152,5 ; 166,8 ; 169,4.

Analyse élémentaire : C₁₂H₁₁C1O₅ ; P.M. = 254

| | C % | H % | Cl % | O % |
|---|---|---|---|---|
| Calculé | 56,60 | 4,35 | 13,92 | 25,13 |
| Trouvé | 56,86 | 4,43 | 13,87 | 25,64 |

### (3) Préparation de l'acide 5,6-O-isopropylidène ascorbique

Dans un ballon tricol de 2 litres muni d'une agitation mécanique d'un réfrigérant et d'un thermomètre, on introduit 100 g d'acide L-ascorbique (0,568 mole) et 1,1 litre d'acétone. On ajoute 2 ml de chlorure d'acétyle (0,028 mole) et porte le milieu vers 40°C. Après 3 h 30 de réaction, on refroidit le milieu réactionnel vers 15°C puis filtre. On reprend le solide sous agitation dans 400 ml d'acétate d'éthyle et le filtre à nouveau. Après séchage sous vide et sur P₂O₅ pendant 24 h, on recueille 80,8 g d'acide 5,6-O-isopropylidène ascorbique (0,374 mole) ; Rdt = 65,8 % sous forme d'un solide blanc cotoneux.
Point de fusion = 202-203°C (Köfler)
Rf. (Gel de Silice : Merck 60 F254) = 0,6 (CH₂Cl₂ : CH₃OH=3:1).
- RMN ¹H (DMSO d6 ; 200 MHz): 1,29(S ; 2xCH₃) ; 3,88-3,95(m; C₆-H); 4,07-4,17(m ; C₆-H) ; 4,26-4,34(m ; C₅-H); 4,72-4,75(m; C₄-H) ; 8,49(large S ; C₃-OH) 11,28(large S ; C₂-OH)
- RMN ¹³C (DMSO d6; 50 MHz): 25,4 ; 25,8 ; 64,9 ; 73,5 ; 74,2 ; 109 ; 118,2 ; 152,3 ; 170,2

Analyse élémentaire : C₉H₁₂O₆ ; P.M. : 216

| | C % | H % | O % |
|---|---|---|---|
| Calculé | 50,00 | 5,56 | 44,44 |
| Trouvé | 49,88 | 5,59 | 44,27 |

### (4) Préparation du 4'-acétoxy férulate de 2-O-[5,6-O-isopropylidène ascorbyle]

Dans un ballon tricol de 500 ml équipé d'un réfrigérant, d'un thermomètre, d'une ampoule d'addition et d'une agitation magnétique, on introduit 7,70 g (113 mmoles) d'imidazole dans 150 ml de dichlorométhane, on ajoute ensuite, goutte à goutte, à température ambiante (20°C), 14,4 g (57 mmoles) du chlorure de l'acide 4-acétoxy férulique en solution dans 120 ml de dichlorométhane.

Après 1 h d'agitation, on ajoute 12,2 g (57 mmoles) d'acide 5,6-O-isopropylidène ascorbique et 60 ml de dichlorométhane. L'agitation est poursuivie pendant 3 h à température ambiante. On évapore le dichlorométhane et reprend le produit brut dans 100 ml de tert-butanol jusqu'à dissolution. On ajoute ensuite 60 ml HCl 1N (60 mmoles) et 300 ml d'eau. Après 30 mn d'agitation, on filtre le précipité obtenu, lave à l'eau et sèche sous vide et sur P₂O₅. On recueille 22,7 g de 4'-acétoxy férulate de 2-O- [5,6-O-isopropylidène ascorbyle] (52 mmoles) : Rdt : 92%.

Après recristallisation dans l'alcool isopropylique on obtient 15,3 g de produit purifié attendu (Rdt. : 62 %).
Point de fusion = 197-198°C (Köfler)
Rf. (Gel de Silice : Merck 60 F254) = 0,63 (CH₂Cl₂ : CH₃OH : NH₄OH à 20% = 78:20:2)
- RMN ¹H (DMSO d6 ; 500 MHz): 1,28(S ; 2xCH₃) ; 2,28(S ; AcO) ; 3,84(S ; OCH₃) ; 3,96(dxd ; C₆-H) ; J₁=8,5 Hz et J₂=6 Hz) ; 4,15(dxd ; C₆-H ; J₁=8,5 Hz et J₃=7 Hz) ; 4,41(dxdxd ; C₅-H ; J₂=6 Hz ; J₃=7 Hz ; J₄=2,5 Hz) ; 5,03 (d ; C₄-H ; J₄=2,5 Hz) ; 6,88 (d ; C=CH-CO ; J₅=16 Hz) ; 7,17 (d ; Ar-H ; J₆=8,5 Hz) ; 7,40(dxd ; Ar-H J₆=8,5 Hz ; J₇=2 Hz) ; 7,60(d ; Ar-H ; J₇=2 Hz) ; 7,82(d ; Ar-CH=C ; J₅=16 Hz) ; 12,94(large S ; C₃-OH) ;
- RMN ¹³C (DMSO d6 ; 50 MHz): 20,5 ; 25,4 ; 25,8 ; 56,1 ; 73,3 ; 75,2 ; 109,3 ; 112,3 ; 112,5 ; 116,6 ; 121,9 ; 123,4 ; 132,9 ; 141,5 ; 146,4 ; 151,3 ; 163,5 ; 163,6 ; 167,9 ; 168,5.

Analyse élémentaire : C₂₁H₂₂O₁₀ ; P.M. = 434

| | C % | H % | O % |
|---|---|---|---|
| Calculé | 58,08 | 5,10 | 36,83 |
| Trouvé | 58,13 | 5,26 | 36,60 |

### (5) Préparation du férulate de 2-O-ascorbyle

Dans un ballon tricol de 250 ml équipé d'un thermomètre, d'un réfrigérant et d'une agitation magnétique, on introduit 6,7 g de 4'-acétoxy férulate 2-O-[5,6-O-isopropylidène ascorbyle] (15,4 mmoles) dans 70 ml d'acétone et ajoute 48 ml d'HCl IN (48 mmoles). On agite le milieu 48 h à température ambiante, puis évapore l'acétone à l'aide d'un évaporateur rotatif On extrait ensuite la phase aqueuse avec 4x50 ml d'acétate d'éthyle puis sèche les phases organiques réunies sur sulfate de sodium. Après concentration jusqu'à un volume d'environ 15 ml d'acétate d'éthyle, on refroidit le milieu et filtre le précipité. On recueille 1,95 g de férulate de 2-O-ascorbyle (5,5 mmoles) ; Rdt = 36 %.
Point de fusion = 157-158°C (Köfler)
Rf. (Gel de Silice : Merck 60 F254) = 0,14(CH₂Cl₂/CH₃OH/NH₄OH (20 %) -78/20/2)
   - RMN ¹H (DMSO d₆ ; 500 MHz): 3,48(m ; 2xC₆-H) ; 3,80(m ; C₅-H+OCH₃) 4,94(d ; C₄-H ; J₁=1,5 Hz) ; 5,07(large S ; C₅-OH et C₆-OH); 6,61(d ; C=CH-CO ; J₂=16 Hz) 6,81(d ; Ar-H ; J₃=8,5 Hz) ; 7,20(dxd ; ArH ; J₃=8,5 Hz et J₄=2 Hz) ; 7,40(d ; Ar-H ; J₄=2 Hz) ; 9,70(S : Ar-OH) ; 12,56(large S ; C₃-OH)
   - RMN ¹³C (DMSO d6 ; 125 MHz): 55,73 (C₁₆) ; 61,73 (C₆) ; 68,60 (C₅) ; 75,43 (C₄) ; 111,60(C₁₁) ; 112,34 (C₂) ; 112,58 (C₈) ; 115,56 (C₁₄) ; 123,59 (C₁₅) ; 125,35(C₁₀) ; 147,32 (C₉) ; 147,96 (C₁₂) ; 149,85 (C₁₃) ; 163,70 (C₃) ; 163,73 (C₇) ; 168,12(C₁)

   La position de la fonction ester sur la vitamine C a été déterminée par RMN en deux dimensions (2D) proton-carbone dans le DMSO d6. Analyse élémentaire : C₁₆H₁₆O₉ ; P.M. = 352

| | C % | H % | O % |
|---|---|---|---|
| Calculé | 54,55 | 4,58 | 40,87 |
| Trouvé | 54,21 | 4,62 | 40,72 |

### EXEMPLE 2 : Préparation du 2-O-cinnamate d'ascorbyle

### (1) Préparation du cinnamate de 2-O-[5,6-O-isopropylidène ascorbyle]

Dans un ballon approprié on dissout 2,70 g (0,04 mole) d'imidazole dans 20 ml de chloroforme puis ajoute, goutte à goutte, en maintenant la température à environ 20°C par un bain d'eau froide, 3,3 g (0,02 mole) du chlorure de l'acide cinnamique en solution dans 40 ml de chloroforme.

Après 1 h d'agitation à température ambiante, on additionne 4,30 g (0,02 mole) d'acide 5,6-O-isopropylidène ascorbique (tel qu'obtenu à l'exemple **1**(3)) en solution dans 50 ml de chloroforme.

Après 2 h d'agitation à température ambiante, le milieu réactionnel est extrait par 100 ml d'une solution de bicarbonate de sodium à 5 %. La phase aqueuse est acidifiée à pH 1 par HCl dilué, et extraite deux fois par 50 ml de dichlorométhane.

Après décantation et séchage sur Na₂SO₄ puis évaporation, on obtient 5,20 g de cinnamate de 2-O-[5,6-O-isopropylidène ascorbyle] (Rdt = 75 %).

Après recristallisation du précipité dans 25 ml d'acétate d'éthyle, on obtient 4,05 g de l'ester attendu (Rdt = 58 %).
Point de fusion = 166-168°C (Köfler)
- RMN ¹H (CDCl₃ ; 500 MHz): 1,40(S ; CH₃); 1,42(S ; CH₃) 4,12(dxd ; C₆-H) ; 4,22(dxd ; C₆-H) ; 4,45 (dxd ; C₅-H) 4,72(d ; C₄-H) ; 6,63(d ; C=CH-CO) ; 7,45(m ; 3xAr-H) ; 7,58(d ; 2xAr-H) ; 7,93(d ; Ar-CH=C) ; 10,29(S ; C=C₃-OH) ;
- RMN ¹³C(CDCl₃, 125 MHz): 25,32 ; 25,51 ; 65,07 ; 73,27 ; 74,38 ; 110,42 ; 114,08 ; 115,28 ; 128,55 ; 128,92 ; 131,52 ; 133,07 ; 150,14 ; 154,67 ; 166,05 ; 166,43.

Analyse élémentaire : C₁₈H₁₈O₇ ; P.M. = 352

| | C % | H % | O % |
|---|---|---|---|
| Calculé | 62,43 | 5,20 | 32,37 |
| Trouvé | 62,24 | 5,17 | 82,19 |

### (2) Préparation du 2-O-cinnamate d'ascorbyle

Dans un ballon tricol de 250 ml on place 4,85 g (0,014 mole) de cinnamate de 2-O-[5,6-O-isopropylidène ascorbyle] dans 100 ml de méthanol, puis ajoute 14 ml d'HCl 1N.

Après 24 h d'agitation à température ambiante, le mélange réactionnel est évaporé à sec, repris dans 50 ml d'eau puis extrait trois fois par 50 ml d'acétate d'éthyle. Après séchage de la phase organique sur Na₂SO₄ et évaporation, on obtient un précipité qui est repris dans 55 ml d'un mélange isopropyl éther/acétone 90/10 et agité 4 h. Après filtration sur verre frité, on obtient 2,80 g de 2-O-cinnamate d'ascorbyle (Rdt = 66 %).
Point de fusion = 144-146°C (Köfler)
- RMN ¹H (CDCl₃ ; 500 MHz): 3,47(m ; 2xC₆-H) ; 3,81(m; C₅H) ; 4,96(d ; C₄-H); 5,1(S ; C₅-OH + C₆OH); 6,80(d : C=CH-CO) ; 7,46(m ; 3xAr-H); 7,80(m ; 2xAr-H+Ar-CH=C); 12,62(S ; =C₃-OH) ;
- RMN ¹³C(CDCl₃ ; 125 Mhz): 61,73 ; 68,82 ; 75,49 ; 112,19 ; 116,29 ; 128,65 ; 128,99 ; 130,94 ; 133,75 ; 146,76 ; 163,36 ; 163,91 ; 168,00.

Analyse élémentaire : C₁₅H₁₄O₇ ; P.M. = 306

| | C % | H % | O % |
|---|---|---|---|
| Calculé | 58,82 | 4,57 | 36,60 |
| Trouvé | 58,74 | 4,69 | 36,61 |

### (3) Préparation du 4'-acétoxy férulate de 2-O-[6-palmitoyl ascorbyle]

Dans un tricol de 250 ml, on introduit 2,95 g d'imidazole dans 130 ml de dichlorométhane. On ajoute ensuite goutte à goutte à température ambiante 5,5 g de chlorure de l'acide 4-acétoxy férulique (obtenu selon l'exemple **1**(2)) en solution dans 50 ml de dichlorométhane.

Après une heure d'agitation, on ajoute 8,95 g de 6-palmitate d'ascorbyle et on agite le mélange pendant 3 h à température ambiante. On évapore le dichlorométhane et on reprend le produit brut dans 30 ml de tert-butanol jusqu'à obtention d'une huile. On additionne alors 25 ml d'acide chlorhydrique 1N puis 100 ml d'eau. Après 30 minutes d'agitation, on filtre le précipité obtenu, lave à l'eau et sèche sous vide sur P₂O₅. On recueille 13,75 g de 4'-acétoxy férulate de 2-O-[6-palmitoyl ascorbyle].

Caractérisation : spectres proton ¹³C 2D conformes à la structure.
- RMN ¹H (CDCl₃) : δ(ppm): 0,873(t, 3H) ; 1,26(m, 26H) ; 1,62(m, 2H) ; 2,32(s, 3H) ; 2,37(t, 2H) ; 3,88(s, 3H) ; 4,28(m, 2H) ; 4,41(dd, 1H) ; 4,86(d, 1H) ; 6,58(d, 1H) ; 7,09(d, 1H) ; 7,15(d, 1H) ;
- RMN ¹³C (CDCl₃) : δ(ppm): 14,12 ; 20,64 ; 22,7 ; 24,86 ; 29,5 ; 31,93 ; 34,1 ; 56,03 ; 64,65 ; 68,13 ; 75,5 ; 111,59 ; 114,4 ; 115,46 ; 122,41 ; 123,6 ; 132,2 ; 142,76 ; 149,65 ; 151,7 ; 155,4 ; 166,47 ; 168,6 ; 173,98.

### EXEMPLES DE COMPOSITION

### EXEMPLE A

On prépare une émulsion du type huile-dans-l'eau en procédant au mélange des ingrédients suivants :

| | |
|---|---|
| 2-O-cinnamate d'ascorbyle | 0,1 % |
| Polyéthylène glycol oxyéthyléné à 50 moles d'OE | 3 % |
| Diglycérol mono stéarate | 3 % |
| Huile de vaseline | 24 % |
| Alcool cétylique | 5 % |
| Eau q.s.p. | 100 % |

### EXEMPLE B

On prépare une émulsion du type huile-dans-l'eau en procédant au mélange des ingrédients suivants :

| | |
|---|---|
| 2-O-férulate d'ascorbyle | 0,1 % |
| Palmitate d'octyle | 10 % |
| Glycérol mono-isostéarate | 4 % |
| Huile de vaseline | 24 % |
| Vitamine E | 1 % |
| Glycérol | 3 % |
| Eau q.s.p. | 100 % |

### EXEMPLE C

On prépare la composition suivante sous forme d'une crème en procédant au mélange des ingrédients suivants :

| | |
|---|---|
| 2-O-férulate d'ascorbyle | 0,1 % |
| Huile de jojoba | 13 % |
| Mélange de méthyl et propyl paraben | 0,05 % |
| Sorbate de potassium | 0,3 % |
| Cyclopentadimethylsiloxane | 10 % |
| Alcool stéarylique | 1 % |
| Acide stéarique | 4 % |
| Stéarate de polyéthylène glycol | 3 % |
| Glycérol | 3 % |
| Eau q.s.p. | 100 % |

Dans cet exemple, le 2-O-férulate d'ascorblyle peut être remplacé par une même quantité du 4'-acétoxy férulate de 2-O-[6-palmitoyl ascorbyle].

Les compositions des exemples A à C sont particulièrement stables dans le temps. Par ailleurs, elles confèrent une protection efficace des cellules épidermiques soumises à l'action des radicaux libres.

## Revendications

1. Mono- et di-esters d'acide cinnamique ou de l'un de ses dérivés et de vitamine C, caractérisés par le fait qu'ils répondent à la formule générale suivante : 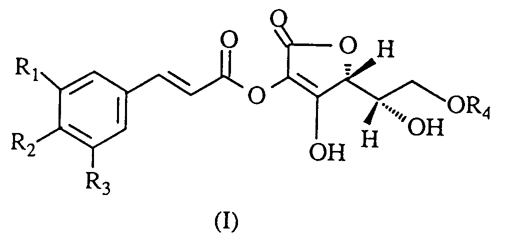 dans laquelle :
R₁, R₂ et R₃, identiques ou différents, représentent un atome d'hydrogène, un radical hydroxy, alkoxy, fluoroalkoxy ou alkylcarbonyloxy, et
R₄ représente un atome d'hydrogène ou -COR₅, R₅ représentant un radical alkyle en C₁-C₂₀ ou le radical de formule :

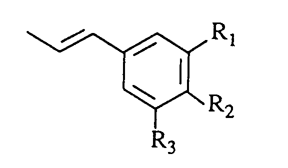

2. Mono- et di-esters selon la revendication 1, caractérisés par le fait que le radical alkoxy est choisi dans le groupe constitué par les radicaux méthoxy, éthoxy et butoxy.

3. Mono- et di-esters selon la revendication 1, caractérisés par le fait que le radical fluoroalkoxy est le radical trifluorométhoxy.

4. Mono- et di-esters selon la revendication 1, caractérisés par le fait que le radical alkylcarbonyloxy est choisi dans le groupe constitué par les radicaux acétoxy, propionyloxy et butyryloxy.

5. Mono- et di-esters selon l'une quelconque des revendications précédentes, caractérisés par le fait qu'ils sont choisis dans le groupe constitué par :
- le 2-O-cinnamate d'ascorbyle,
- le 2-O-férulate d'ascorbyle,
- le 2-O-caféate d'ascorbyle,
- le 2-O-sinapate d'ascorbyle, et
- le 4'-acétoxy férulate de 2-O-[6-palmitoyl ascorbyle].

6. Procédé de préparation des mono-esters d'acide cinnamique ou de l'un de ses dérivés et de vitamine C de formule (I) selon la revendication 1 dans laquelle R₄ représente un atome d'hydrogène, caractérisé par le fait qu'il consiste :
(a) à préparer dans un premier temps l'acide 5,6-O-isopropylidène ascorbique de formule : 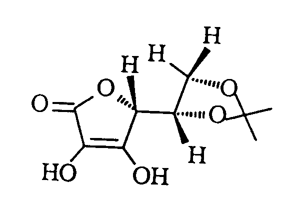 par réaction de l'acide ascorbique dans l'acétone en présence de chlorure d'acétyle en vue de protéger les fonctions hydroxy en positions 5 et 6 de l'acide ascorbique,
(b) à faire réagir l'acide 5,6-O-isopropylidène ascorbique avec un chlorure d'acide de formule : 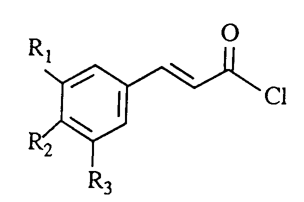 dans laquelle :
R₁, R₂ et R₃, sont tels que définis à la revendication 1, la réaction étant effectuée dans un solvant organique en présence d'imidazole, et
(c) à déprotéger les fonctions dihydroxy en positions 5 et 6 dans l'acétone en présence d'HCl 1N à 3N.

7. Procédé de préparation des di-esters d'acide cinnamique ou de l'un de ses dérivés et de vitamine C de formule (I) selon la revendication 1 dans laquelle R₄ représente le radical -COR₅ caractérisé par le fait qu'il consiste :
(a) à préparer dans un premier temps un ester en position 6 de l'acide ascorbique de formule : 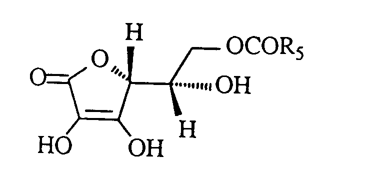 dans laquelle :
R₅ a la même signification qu'à la revendication 1, par réaction d'acylation, et
(b) à faire réagir l'ester d'ascorbyle en position 6 avec un chlorure d'acide de formule : 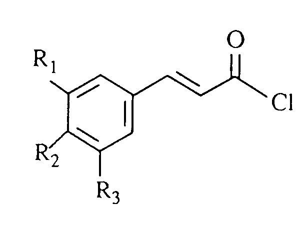 dans laquelle :
R₁, R₂ et R₃ sont tels que définis à la revendication 1, la réaction étant effectuée dans un solvant organique en présence d'imidazole.

8. Procédé selon les revendications 6 et 7, caractérisé par le fait que le solvant organique de l'étape (b) est le dichlorométhane.

9. Procédé selon les revendications 6 à 8, caractérisé par le fait que lorsque l'un au moins des radicaux R₁, R₂ et R₃ est un radical hydroxy, celui-ci est préalablement protégé par formation d'un radical acétoxy, puis déprotégé dans l'acétone en présence d'HCl 1N à 3N.

10. Composition à base de corps gras, caractérisée par le fait qu'elle contient au moins un mono- ou di-ester d'acide cinnamique ou de l'un de ses dérivés et de vitamine C selon l'une quelconque des revendications 1 à 5, ou obtenu selon l'une quelconque des revendications 6 à 9.

11. Composition cosmétique, pharmaceutique ou alimentaire, caractérisée par le fait qu'elle contient au moins un mono- ou di-ester d'acide cinnamique ou de l'un de ses dérivés et de vitamine C selon l'une quelconque des revendications 1 à 5, ou obtenu selon l'une quelconque des revendications 6 à 9, en une proportion comprise entre 0,01 et 5 % en poids par rapport au poids total de la composition.

12. Composition selon la revendication 11, caractérisée par le fait que le mono-ou di-ester d'acide cinnamique ou de l'un de ses dérivés et de vitamine C est choisi dans le groupe constitué par :
- le 2-O-cinnamate d'ascorbyle,
- le 2-O-férulate d'ascorbyle,
- le 2-O-caféate d'ascorbyle,
- le 2-O-sinapate d'ascorbyle, et
- le 4'-acétoxy férulate de 2-O-[6-palmitoyl ascorbyle].

13. Composition selon les revendications 11 et 12, caractérisée par le fait qu'elle se présente sous forme d'une crème cosmétique ou dermo-pharmaceutique destinée à la protection de l'oxydation des lipides de la peau.

## Claims

1. Mono- and diesters of cinnamic acid or of one of its derivatives and of vitamin C, characterized in that they correspond to the following general formula: 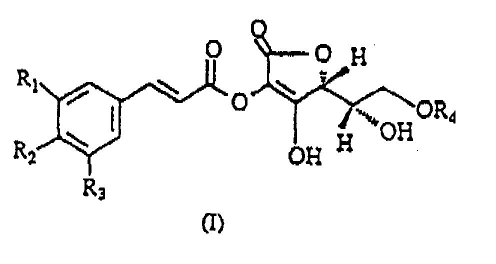 in which:
R₁, R₂ and R₃, which are identical or different, represent a hydrogen atom or a hydroxyl, alkoxy, fluoroalkoxy or alkylcarbonyloxy radical, and
R₄ represents a hydrogen atom or -COR₅, R₅ representing a C₁-C₂₀ alkyl radical or the radical of formula:

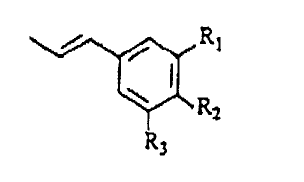

2. Mono- and diesters according to Claim 1, characterized in that the alkoxy radical is chosen from the group consisting of the methoxy, ethoxy and butoxy radicals.

3. Mono- and diesters according to Claim 1, characterized in that the fluoroalkoxy radical is the trifluoromethoxy radical.

4. Mono- and diesters according to Claim 1, characterized in that the alkylcarbonyloxy radical is chosen from the group consisting of the acetoxy, propionyloxy and butyryloxy radicals.

5. Mono- and diesters according to any one of the preceding claims, characterized in that they are chosen from the group consisting of:
- ascorbyl 2-O-cinnamate,
- ascorbyl 2-O-ferulate,
- ascorbyl 2-O-caffeate
- ascorbyl 2-O-sinapate, and
- 2-O-[6-palmitoylascorbyl] 4'-acetoxyferulate.

6. Process for the preparation of the monoesters of cinnamic acid or of one of its derivatives and of vitamin C of formula (I) according to Claim 1 in which R₄ represents a hydrogen atom, characterized in that it consists:
(a) in preparing, in a first step, 5,6-O-isopropylideneascorbic acid of formula: 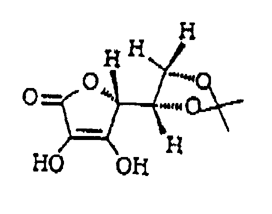 by reaction of ascorbic acid in acetone in the presence of acetyl chloride, for the purpose of protecting the hydroxyl functional groups in the 5- and 6-positions of ascorbic acid,
(b) in reacting 5,6-O-isopropylideneascorbic acid with an acid chloride of formula: 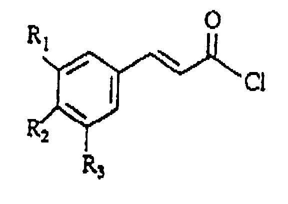 in which:
R₁, R₂ and R₃ are as defined in Claim 1, the reaction being carried out in an organic solvent in the presence of imidazole, and
(c) in deprotecting the dihydroxyl functional groups in the 5- and 6-positions in acetone in the presence of 1N to 3N HCl.

7. Process for the preparation of the diesters of cinnamic acid or of one of its derivatives and of vitamin C of formula (I) according to Claim 1 in which R₄ represents the -COR₅ radical, characterized in that it consists:
(a) in preparing, in a first step, an ester in the 6-position of ascorbic acid of formula: 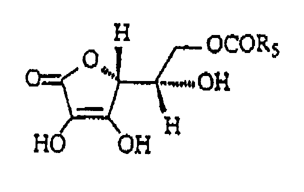 in which:
R₅ has the same meaning as in Claim 1, by an acylation reaction, and
(b) in reacting the ascorbyl ester in the 6-position with an acid chloride of formula: 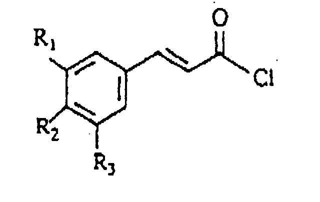 in which:
R₁, R₂ and R₃ are as defined in Claim 1, the reaction being carried out in an organic solvent in the presence of imidazole.

8. Process according to Claims 6 and 7, characterized in that the organic solvent of stage (b) is dichloromethane.

9. Process according to Claims 6 to 8, characterized in that, when at least one of the R₁, R₂ and R₃ radicals is a hydroxyl radical, the latter is protected beforehand by formation of an acetoxy radical and then deprotected in acetone in the presence of 1N to 3N HCl.

10. Composition based on a fatty substance, characterized in that it comprises at least one monoor diester of cinnamic acid or of one of its derivatives and of vitamin C according to any one of Claims 1 to 5 or obtained according to any one of Claims 6 to 9.

11. Cosmetic, pharmaceutical or food composition, characterized in that it comprises at least one monoor diester of cinnamic acid or of one of its derivatives and of vitamin C according to any one of Claims 1 to 5 or obtained according to any one of Claims 6 to 9 in a proportion of between 0.01 and 5% by weight with respect to the total weight of the composition.

12. Composition according to Claim 11, characterized in that the mono- or diester of cinnamic acid or of one of its derivatives and of vitamin C is chosen from the group consisting of:
- ascorbyl 2-O-cinnamate,
- ascorbyl 2-O-ferulate,
- ascorbyl 2-O-caffeate
- ascorbyl 2-O-sinapate, and
- 2-O-[6-palmitoylascorbyl] 4'-acetoxyferulate.

13. Composition according to Claims 11 and 12, characterized in that it is provided in the form of a cosmetic or dermopharmaceutical cream intended for the protection from oxidation of the lipids of the skin.

## Patentansprüche

1. Mono- und Diester der Zimtsäure oder von deren Derivaten mit Vitamin C, dadurch gekennzeichnet, daß sie folgender allgemeiner Formel entsprechen: 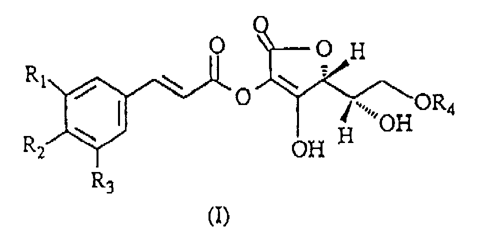 in der
R₁, R₂ und R₃, gleich oder verschieden, ein Wasserstoffatom, einen Hydroxy-, Alkoxy, Fluoralkoxy- oder Alkylcarbonyloxyrest bedeuten, und
R₄ ein Wasserstoffatom oder -COR₅ bedeutet, wobei R₅ einen C₁-C₂₀-Alkylrest oder einen Rest der Formel 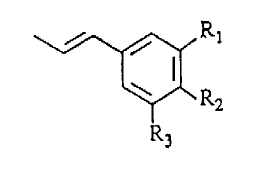 bedeutet.

2. Mono- und Diester nach Anspruch 1, dadurch gekennzeichnet, daß der Alkoxyrest aus der Gruppe, bestehend aus Methoxy-, Ethoxy- und Butoxyresten, ausgewählt ist.

3. Mono- und Diester nach Anspruch 1, dadurch gekennzeichnet, daß der Fluoralkoxyrest der Trifluormethoxyrest ist.

4. Mono- und Diester nach Anspruch 1, dadurch gekennzeichnet, daß der Alkylcarbonyloxyrest aus der Gruppe, bestehend aus Acetoxy-, Propionyloxy- und Butyryloxyresten, ausgewählt ist,

5. Mono- und Diester nach einem der vorstehenden Ansprüche, dadurch gekennzeichnet, daß sie ausgewählt sind aus der Gruppe:
- 2-O-Ascorbylcinnamat,
- 2-O-Ascorbylferulat,
- 2-O-Ascorbylcafeat,
- 2-O-Ascorbylsinapat, und
- 2-O-[6-Palmitoylascorbyl]-4'-acetoxyferulat.

6. Verfahren zur Herstellung von Monoestern der Zimtsäure oder eines ihrer Derivate mit Vitamin C der Formel (I) nach Anspruch 1, in der R₄ ein Wasserstoffatom bedeutet, dadurch gekennzeichnet, daß man;
(a) in einem ersten Schritt 5,6-O-Isopropylidenascorbinsäure der Formel 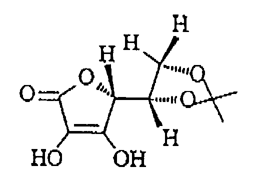 durch Umsetzung von Ascorbinsäure in Aceton in Anwesenheit von Acetylchlorid herstellt, um die Hydroxyfunktionen in den Stellungen 5 und 6 der Ascorbinsäure zu schützen,
(b) die 5,6-O-Isopropylidenascorbinsäure mit einem Säurechlorid der Formel 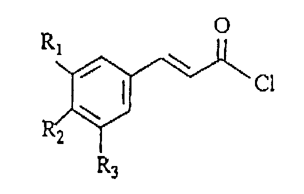 zur Umsetzung bringt, in der
R₁,R₂ und R₃ die in Anspruch 1 angegebene Bedeutung haben, wobei die Umsetzung in einem organischen Lösungsmittel in Anwesenheit von Imidazol durchgeführt wird, und
(c) die Hydroxyfunktionen in den Stellungen 5 und 6 in Aceton in Gegenwart von HCl (1N bis 3N) entschützt.

7. Verfahren zur Herstellung von Diestern der Zimtsäure oder eines ihrer Derivate mit Vitamin C der Formel (I) nach Anspruch 1, in der R₄ den -COR₅-Rest bedeutet, dadurch gekennzeichnet, daß man
(a) zuerst einen Ester in 6-Stellung der Ascorbinsäure der Formel 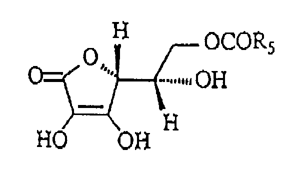 durch Acylierung herstellt, in der R₅ die in Anspruch 1 angegebene Bedeutung hat, und
(b) den Ascorbylester in 6-Stellung mit einem Säurechlorid der Formel 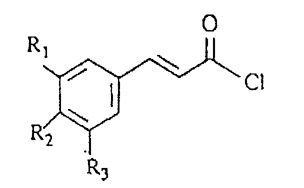 zur Umsetzung bringt,
R₁, R₂ und R₃ die in Anspruch 1 angegebene Bedeutung haben, und die Umsetzung in einem organischen Lösungsmittel in Gegenwart von Imidazol durchgeführt wird.

8. Verfahren nach den Ansprüchen 6 und 7, dadurch gekennzeichnet, daß das organische Lösungsmittel der Stufe (b) Dichlormethan ist.

9. Verfahren nach den Ansprüchen 6 bis 8, dadurch gekennzeichnet, daß, wenn mindestens einer der Reste R₁, R₂ und R₃ ein Hydroxyrest ist, dieser vorher durch Bildung eines Acetoxyrestes geschützt wird, und anschließend in Aceton in Anwesenheit von HCl (1N bis 3N) die Schutzgruppe entfernt wird.

10. Zubereitung auf Fettbasis, dadurch gekennzeichnet, daß sie mindestens einen Mono- oder Diester der Zimtsäure oder eines ihrer Derivate mit Vitamin C gemäß einem der Ansprüche 1 bis 5 oder erhalten nach einem der Ansprüche 6 bis 9 enthält.

11. Kosmetische Zubereitung, pharmazeutische Zubereitung oder Nährstoffzubereitung, dadurch gekennzeichnet, daß sie mindestens einen Mono- oder Diester der Zimtsäure oder eines ihrer Derivate mit Vitamin C nach einem der Ansprüche 1 bis 5 oder gemäß einem der Ansprüche 6 bis 9 erhalten in einem Verhältnis zwischen 0,01 und 5 Gew.-%, bezogen auf das Gesamtgewicht der Zubereitung, enthält.

12. Zubereitung nach Anspruch 11, dadurch gekennzeichnet, daß der Mono- oder Diester der Zimtsäure oder eines ihrer Derivate mit Vitamin C ausgewählt ist aus der Gruppe:
- 2-O-Ascorbylcinnamat,
- 2-O-Ascorbylferulat,
- 2-O-Ascorbylcafeat,
- 2-O-Ascorbylsinapat, und
- 2-O-[6-Palmitoylascorbyl]-4'-acetoxyferulat.

13. Zubereitung nach den Ansprüchen 11 und 12, dadurch gekennzeichnet, daß sie in Form einer kosmetischen oder dermo-pharmazeutischen Creme zum Schutz vor Oxidation von Lipiden der Haut vorliegt.
